# EUROPEAN PATENT APPLICATION

(11) **EP 4 729 056 A1**
(43) Date of publication of application: **22.04.2026**
(21) Application number: 23851280.0
(22) Date of filing: 06.05.2023
(51) Int. Cl.: A61K 31/337, A61K 9/00, A61K 9/107, A61K 47/12, A61K 47/44, A61K 47/24, A61P 35/00

(54) **SELF-EMULSIFIABLE DOCETAXEL COMPOSITION FOR INJECTION AND PREPARATION METHOD THEREFOR**

(71) Applicant: BEIJING DELIINJE PHARM. TECHNOLOGY CO., LTD., Beijing 102102 (CN)
(72) Inventor: WU, Cuishuan, Beijing 102102 (CN); ZHANG, Dan, Beijing 102102 (CN); SUN, Yamei, Beijing 102102 (CN)
(74) Representative: Hofstetter, Schurack & Partner
(86) International application number: PCT/CN2023/092534
(87) International publication number: WO 2024/032050

(57) **Abstract**

The present invention relates to a self-emulsifiable docetaxel composition for injection and a preparation method therefor. The present invention belongs to the field of pharmaceutical preparations. More specifically, the present invention relates to a self-emulsifiable docetaxel composition for injection and a preparation method therefor.

## Description

### Technical Field

The invention belongs to the field of pharmaceutical preparations. More particularly, the present disclosure relates to a self-emulsifiable docetaxel composition for injection and a preparation method thereof.

### Background

Tumors are serious intractable diseases threatening the life and health of humans, and the treatment of tumors has been paid close attention all over the world. Currently, tumors gradually replace cardiovascular and cerebrovascular diseases to become the globally first killer. According to the latest report about cancer in China, 2019 issued by the National Cancer Center, the incidence and morality of malignant tumors in 2015 are about 3.929 million people and about 2.339 million people; on average, more than ten thousand people were diagnosed with cancer per day, and 7.5 people were diagnosed with cancer per minute. Over the last 10 years, the incidence and morality of malignant tumors have remained approximately increase of 3.9% and 2.5% in China each year.

Docetaxel is one of the most important and successful medicaments for treating cancer such as non-small cell lung cancer, breast cancer, prostate cancer, gastric cancer and the like, and has significant clinical requirements and huge market. Although docetaxel has a remarkable therapeutic effect, docetaxel is hardly soluble in water, while it is soluble in organic solvents such as methanol and chloromethane. Docetaxel injection of Sanofi-Aventis was approved by the National Medical Products Administration under the brand name Taxotere^{®} in 2008. To solve the solubility issue of docetaxel, a large amount of the surfactant Tween-80 (also known as polysorbate 80) is used as a solubilizer in the widely clinically used docetaxel injections, including Taxotere^{®}, and a special solvent for reconstitution attached to lyophilized docetaxel preparations for injection is also an ethanol-containing Tween-80 solution.

A large number of studies have shown that clinical applications are greatly affected by severe allergic reactions, such as drug rashes, shortness of breath, bronchospasm, hypotension, hemolysis and fluid retention, in some patients after intravenous administration of an injection containing Tween-80. Thus, in addition to bone marrow toxicity (neutropenia, leukopenia, anemia) and liver toxicity caused by docetaxel itself, the major adverse effects of currently clinically used docetaxel injections, including Taxotere^{®}, include allergic reactions caused by Tween-80. For example, the package insert of Taxotere^{®} clearly indicates as "WARNING" on its first page "Contraindicated if history of severe hypersensitivity reactions to TAXOTERE or to drugs formulated with polysorbate 80", and "Docetaxel must be used under the guidance of a physician who is experienced in the use of chemotherapeutic drugs for cancer. Due to the possibility of severe allergic reactions, appropriate emergency medical facilities should be available, and close monitoring of key functional indicators is recommended during injection" and "Patients should take prophylactic medication before receiving docetaxel treatment to reduce the incidence and severity of fluid retention and to reduce the severity of allergic reactions, including oral corticosteroids, such as dexamethasone at 16mg per day (8mg BID), starting one day before docetaxel injection and continuing for 3 days" under "PRECAUTIONS". That is, in order to alleviate the severe allergic reactions caused by Tween-80, patients need to take glucocorticoids such as dexamethasone in advance. Therefore, since the docetaxel injections contain the surfactant Tween-80, the safety and compliance of the currently available docetaxel injections are poor in clinical use.

In addition, even though they have contained Tween-80, the currently available docetaxel preparations have problems in terms of stability, resulting in very cumbersome and inconvenient procedures in clinical use.

For example, docetaxel injection of Sanofi-Aventis was approved by the National Medical Products Administration under the brand name Taxotere^{®} in 2008. Taxotere^{®} is a concentrate solution containing Tween-80 in the form of a yellow to brown viscous liquid, which is equipped with a solvent consisting of 95% ethanol and water for injection. Its use method comprises aseptically withdrawing the viscous liquid containing docetaxel and Tween-80 with a syringe, injecting it into the equipped solvent, manually mixing by inversion for at least 45 seconds without shaking, standing at room temperature for 5 min, and checking whether the solution is uniform and clear, to give a "pre-injection". The pre-injection is then aseptically withdrawn with a syringe and injected into 5% glucose solution or 0.9% saline to form an "injection" for intravenous infusion. The "pre-injection" should be used immediately after formulation, and could be stored stably for up to 8 hours at 2-8°C or at room temperature, and the "injection" must be used within 4 hours. In addition, both the "pre-injection" and the "injection" may form precipitates, and should be inspected before use. If the precipitate is visually observed, they must be discarded without use.

As another example, docetaxel injection manufactured by Eagle Pharmaceuticals, Inc. is a concentrate solution containing Tween-80 but not containing ethanol. In clinical use, the concentrate solution should be withdrawn from the vial with syringe and injected into 250ml of 0.9% sodium chloride solution or 5% glucose solution to form the final injection for intravenous infusion. The final injection can keep stable for only 24 hours at 2°C to 25°C. Moreover, before administering the final injection to a patient, it shall also be checked and must be discarded without use once precipitate is visually observed.

As docetaxel preparations currently available in the market in China and abroad comprise Tween-80 as a solubilizer, which is considered to be related to severe allergic reactions, a novel docetaxel preparation without Tween-80 has been developed in all countries to avoid the obvious toxic and adverse effects brought by Tween-80 and improve the compliance. However, since Tween-80 is a key component for maintaining the stability of the preparations, it is a big challenge to develop a docetaxel drug delivery system free of Tween-80, which has good physical and chemical stability and does not have significant toxic and adverse effects.

Several new docetaxel formulations are currently tested in the clinical trials. For example, the docetaxel micelle preparation (Nanoxel-PM) developed by Samyang Holdings Biopharmaceutical dispersions, Republic of Korea uses mPEG-PLA as the polymeric micelle material, and is currently undergoing a multicenter, open-label, randomized, and crossover bioequivalence study. In addition, the docetaxel albumin nanoparticles (Nab^{®} Docetaxel, ABI-008) developed by Celgene, the USA, the docetaxel lipid suspension developed by INTA company, India (DoceAqualip, NCT 01957995), and the docetaxel injection (DICN, lipid and polymer-containing automatic dispersion system) not containing Tween-80 developed by Sun Pharma, India, and the like all have entered the clinical trials.

These new preparations can reduce the toxic and adverse effect caused by Tween-80, but also has new defects: 1) new solubilizers or toxic substances are introduced, and the new introduced substances bring new toxic and adverse effects. For example, some preparations contain polyoxyethylene castor oil, which also causes allergic reactions, bronchospasm, tachypnea, tiredness, hypotension and the like; 2) new non-medicinal polymers are introduced, and the source and toxicity problems of those polymers influence the development prospect of the preparations; and 3) the preparation process is relatively complex, production in industrial scale is difficult, and the poor reproducibility of the process, residual organic solvent and the like are problematic.

In order to overcome the above defects, the Chinese patent ZL201010268940.0 disclosed a drug delivery system for poorly soluble drugs, which was prepared using phospholipid and injectable oil as main carriers by dissolving the poorly soluble drugs in a solvent or injectable oil or a mixture thereof, adding phospholipid and other components, and stirring to make the mixture uniform to give a liquid composition. The system can be used to deliver a variety of poorly soluble drugs, including docetaxel. The composition can be diluted with glucose injection before use to form an emulsion injection through self-emulsification. The formed emulsion injection is physically stable, has good dispersibility, and is convenient for clinical use. However, it is found that although the docetaxel liquid composition disclosed in the Chinese patent ZL201010268940.0 avoided the adverse reactions of Tween-80, it was not chemically stable enough. Upon storage, its impurities were above the specified limits, and the content of the Impurity e (4-Epicetaxel) was increased. Said Impurity e has the following structure:

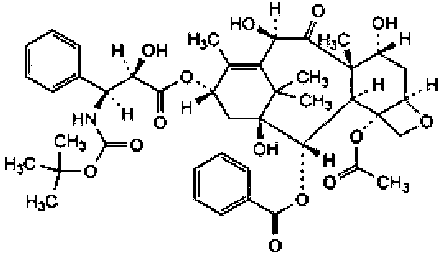

### Impurity E

Impurity E causes tumor cells to develop resistance to the chemotherapeutic agents including docetaxel. For this reason, the United States Pharmacopoeia particularly prescribes a limit on the content of this substance. Minimizing the level of this impurity has been one of the goals in docetaxel drug development. Therefore, there is a need to provide a novel docetaxel pharmaceutical composition without Tween-80, which is capable of ensuring good dissolution of docetaxel and good self-emulsification ability upon dilution with an aqueous vehicle for injection, and improving the physical stability of an emulsion formed upon dilution and the chemical stability of the composition itself, particularly reducing the production of impurities.

The inventors have surprisingly found that the addition of a specific amount of citric acid to a docetaxel pharmaceutical composition can solve the problem.

### Description of Invention

In the first aspect, the present disclosure provides a self-emulsifiable docetaxel composition for injection. The composition has good chemical stability and good self-emulsification ability upon dilution with an injectable aqueous vehicle. The emulsion formed by dilution of the composition has good physical stability and can be stored for a long time. These characteristics offer significant advantages to the composition of the present disclosure in terms of transportation and storage.

More specifically, the docetaxel composition for injection consists of docetaxel, an injectable oil, a phospholipid, a low-molecular-weight alcohol and citric acid, wherein citric acid accounts for 0.05%-0.2% w/w of the total weight of the composition.

In one embodiment of this aspect, docetaxel accounts for 0.1%-5% w/w of the total weight of the composition of the present disclosure. Preferably, docetaxel accounts for 1% to 3.5% w/w, such as 1.5%-3.5% w/w, 1.5%-3% w/w, 2%-2.5% w/w of the total weight of the composition of the present disclosure. More preferably, docetaxel accounts for 2.25% w/w of the total weight of the composition of the present disclosure.

In another embodiment of this aspect, the injectable oil accounts for 5%-20% w/w of the total weight of the composition of the present disclosure. Preferably, the injectable oil accounts for 6%-15% w/w, for example 8%-15% w/w or 10%-12% w/w, of the total weight of the composition of the present disclosure. More preferably, the injectable oil accounts for 11.25% w/w of the total weight of the composition of the present disclosure.

In another embodiment of this aspect, the phospholipid accounts for 25%-40% w/w of the total weight of the composition of the present disclosure. Preferably, the phospholipid accounts for 30%-40% w/w, for example 30%-35% w/w, of the total weight of the composition of the present disclosure. More preferably, the phospholipid accounts for 33.74% w/w of the total weight of the composition of the present disclosure.

In another embodiment of this aspect, citric acid accounts for 0.05%-0.1% w/w of the total weight of the composition of the present disclosure. Preferably, citric acid accounts for 0.1% w/w of the total weight of the composition of the present disclosure.

In the composition of the present disclosure, the low-molecular-weight alcohol accounts for the balance, except docetaxel, the injectable oil, the phospholipid and citric acid.

For example, in a preferred embodiment of this aspect, the composition of the present disclosure consists of:

| | |
|---|---|
| Docetaxel | 0.1%-5% w/w |
| Injectable oil | 5%-20% w/w |
| Phospholipid | 25%-40% w/w |
| Citric acid | 0.05%-0.2% w/w |
| Low-molecular-weight alcohol | make up to 100% w/w. |

In another preferred embodiment of this aspect, the composition of the present disclosure consists of:

| | |
|---|---|
| Docetaxel | 1%-3.5% w/w |
| Injectable oil | 6%-15% w/w |
| Phospholipid | 25%-40% w/w |
| Citric acid | 0.05%-0.1% w/w |
| Low-molecular-weight alcohol | make up to 100% w/w. |

In another preferred embodiment of this aspect, the composition of the present disclosure consists of:

| | |
|---|---|
| Docetaxel | 2.25% w/w |
| Injectable oil | 11.25% w/w |
| Phospholipid | 33.74% w/w |
| Citric acid | 0.1% w/w |
| Low-molecular-weight alcohol | make up to 100% w/w. |

Preferably, in the above embodiments, the injectable oil is soybean oil.

Preferably, in the above embodiments, the phospholipid is egg yolk lecithin.

Preferably, in the above embodiments, the low-molecular-weight alcohol is ethanol.

Most preferably, the docetaxel self-emulsifiable composition for injection of the present disclosure consists of:

| | |
|---|---|
| Docetaxel | 2.25% w/w |
| Injectable soybean oil | 11.25% w/w |
| Egg yolk lecithin | 33.74% w/w |
| Citric acid | 0.05%, 0.1% or 0.2% w/w |
| Ethanol | make up to 100% w/w. |

The composition of the present disclosure is a self-emulsifiable system, which is diluted by an aqueous vehicle for injection before clinical use to spontaneously form a fat emulsion with a droplet size of 200-700nm, preferably 300-500nm. The fat emulsion can be directly administered by injection to a patient.

The composition of the present disclosure can, upon being diluted with an aqueous vehicle for injection, be administered alone or in combination with other drugs such as cisplatin, 5-fluorouracil, etc. to treat cancer.

In the second aspect, the present disclosure provides a method for preparing the composition of the present disclosure, comprising:
(1) Dissolving docetaxel and citric acid in the low-molecular-weight alcohol, and then sequentially adding with the phospholipid and the injectable oil to form a uniform solution; and
(2) Sterilizing the uniform solution obtained in step (1) by filtration, and filling the solution into a container to give the docetaxel composition of the present disclosure.

In the third aspect, the present disclosure provides a pharmaceutical product comprising the docetaxel composition of the present disclosure.

In one embodiment of this aspect, the pharmaceutical product is an emulsion. Preferably, the emulsion consists of the docetaxel composition of the present disclosure and an aqueous vehicle for injection, preferably selected from 5% glucose injection, 0.9% sodium chloride injection, glucose and sodium chloride injection, water for injection, and any mixture thereof. More preferably, the emulsion has a droplet size of 200-700nm, preferably 300-500 nm.

In a fourth aspect, the present disclosure provides the use of the docetaxel composition of the present disclosure in the preparation of a medicament such as emulsion for the treatment of cancer.

The terms "emulsion" and "fat emulsion" as described herein may be used interchangeably, and are also referred to as "the emulsion of the present disclosure". The emulsion of the present disclosure can be directly administered by injection to a patient. The administration by injection may be intravenous, subcutaneous, intramuscular, intrathecal injection, preferably intravenous injection, e.g. intravenous bolus injection or intravenous infusion.

In the fifth aspect, the present disclosure provides a method of treating cancer, comprising intravenously administering a therapeutically effective amount of the emulsion of the present disclosure to a patient with cancer.

Docetaxel can be administered to a patient in an amount of 60 mg/m² body surface area to 75 mg/m² body surface area. The specific amount will depend on the cancer to be treated, the health of the patient, concomitant medication, and other factors, and can be readily determined by the clinician.

### Definitions

Docetaxel as used herein refers to a compound of the structural formula:

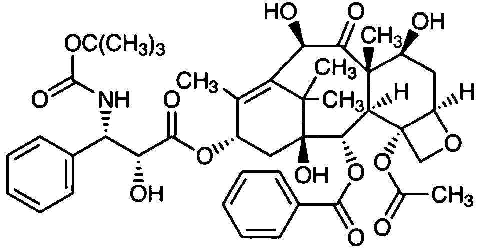

Docetaxel can be in the form of anhydride or hydrate such as trihydrate. In the present disclosure, docetaxel used in the composition is in the form of anhydride. The amount of docetaxel is indicated in anhydrous form, regardless of the form in which it is used.

The injectable oil as described herein is selected from one or more of soybean oil, fish oil, olive oil, tea oil, oleic acid, sesame oil, safflower oil, castor oil, peanut oil, palm oil, and medium chain fatty acid triglycerides. Preferably, the injectable oil as described herein is soybean oil.

The phospholipid as described herein is selected from one or more of egg yolk lecithin, soybean phospholipid, hydrogenated soybean phospholipid, dierucoyl phosphatidylcholine, 1,2-dimyristoyl-sn-glycero-3-phosphocholine, phosphatidyl ethanolamine (Cephalin), phosphatidyl serine, phosphatidyl glycerol, diphosphatidyl glycerol (Cardiolipin), phosphatidyl inositol and sphingomyelin. Preferably, the phospholipid as described herein is selected from one or more of egg yolk lecithin, soybean phospholipid, phosphatidyl ethanolamine, phosphatidyl serine. More preferably, the phospholipid as described herein is egg yolk lecithin.

The low-molecular-weight alcohol as described herein refers to C₁₋₆ alkanol, that is, a straight- or branched-chain saturated hydrocarbon group having 1 to 6 carbon atoms substituted with one or more hydroxyl groups. Preferably, the low-molecular-weight alcohol as described herein is selected from one or more of ethanol, ethylene glycol, propylene glycol, glycerol, isopropanol, n-butanol, isobutanol and pentanol. More preferably, the low-molecular-weight alcohol as described herein is ethanol.

Citric acid as described herein has the following structure:

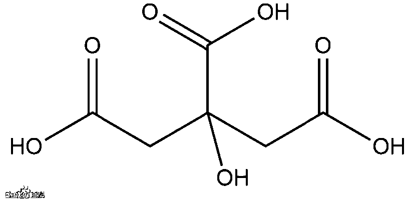

Citric acid may be in the form of anhydride or hydrate such as monohydrate. In the present disclosure, citric acid used in the composition is anhydride. The amount of citric acid is indicated in anhydrous form, regardless of the form in which it is used.

The percentages of the individual components indicated in the present disclosure are weight percentages based on the total weight of the composition, expressed as % w/w, unless specified otherwise.

As used herein, the terms "comprise", "contain", "include" and their variants mean that the technical solution in connection with such terms includes, but is not limited to, the specified components or steps. These terms encompass and preferably are those embodiments that are formed solely from the specified components or steps, that is, those embodiments that "consist of" the specified components or steps.

The term "room temperature" as used herein means 10-30°C.

The term "medium chain fatty acid triglyceride" as used herein is also known as Medium Chain Triglyceride (MCT), and refers to a triglyceride of fatty acid having a saturated or unsaturated carbon chain of 6-12 carbon atoms. MCT are widely used in injectable emulsions. Preferably, MCT refers to saturated caprylic triglyceride, or saturated capric triglyceride, or saturated caprylic/capric triglyceride. MCT is commercially available, for example, under the trade names Miglyol 210, Miglyol 812, Crodamol GTCC, and the like.

The term "ethanol" as used herein refers to CH₃CH₂OH. The ethanol as used herein is commercially available, preferably is 95% ethanol or absolute ethanol (i.e., 99.5% ethanol), more preferably is absolute ethanol.

The term "cancer" as used herein is, for example, lung cancer such as non-small cell lung cancer and small cell lung cancer; breast cancer such as locally advanced or metastatic breast cancer, HER2-positive metastatic breast cancer, and node-positive breast cancer; prostate cancer such as hormone-refractory metastatic prostate cancer; gastric cancer such as advanced gastric adenocarcinoma; ovarian cancer; head and neck cancer; pancreatic cancer; melanoma; soft tissue sarcoma, and the like.

The term "therapeutically effective amount" as used herein refers to an amount of docetaxel that alleviates the symptoms of cancer, reduces the size of a tumor, prolongs patient survival, and/or improves life quality in a patient.

The term "aqueous vehicle for injection" as used herein refers to an aqueous vehicle for injection that can be used to dilute a drug, and can be selected from, for example, 5% glucose injection, 0.9% sodium chloride injection, glucose and sodium chloride injection, water for injection, and any mixtures thereof.

### Embodiments

Embodiment 1. A self-emulsifiable docetaxel composition for injection, consisting of docetaxel, an injectable oil, a phospholipid, a low-molecular-weight alcohol and citric acid, wherein citric acid accounts for 0.05%-0.2% w/w based on the total weight of the composition.

Embodiment 2. The composition according to Embodiment 1, wherein the amounts of docetaxel, the injectable oil, the phospholipid, the low-molecular-weight alcohol and citric acid in the composition are as follows:

| | |
|---|---|
| Docetaxel | 0.1%-5% w/w |
| Injectable oil | 5%-20% w/w |
| Phospholipid | 25%-40% w/w |
| Citric acid | 0.05%-0.2% w/w |
| Low-molecular-weight alcohol | make up to 100% w/w. |

Embodiment 3. The composition according to Embodiment 1 or 2, wherein docetaxel accounts for 1%-3.5% w/w, for example, 1.5%-3.5% w/w, 1.5%-3% w/w, 2%-2.5% w/w, based on the total weight of the composition.

Embodiment 4. The composition according to any one of Embodiments 1 to 3, wherein the injectable oil accounts for 6%-15% w/w, for example, 8%-15% w/w or 10%-12% w/w, based on the total weight of the composition.

Embodiment 5. The composition according to any one of Embodiments 1 to 4, wherein the phospholipid accounts for 30%-40% w/w, for example, 30%-35% w/w, based on the total weight of the composition.

Embodiment 6. The composition according to any one of Embodiments 1 to 5, wherein citric acid accounts for 0.05%-0.1% w/w, preferably 0.1% w/w, based on the total weight of the composition.

Embodiment 7. The composition according to Embodiment 1, wherein the composition consists of:

| | |
|---|---|
| Docetaxel | 1%-3.5% w/w |
| Injectable oil | 6%-15% w/w |
| Phospholipid | 25%-40% w/w |
| Citric acid | 0.05%-0.1% w/w |
| Low-molecular-weight alcohol | make up to 100% w/w. |

Embodiment 8. The composition according to any one of Embodiments 1 to 7, wherein the injectable oil is selected from one or more of soybean oil, fish oil, olive oil, tea oil, oleic acid, sesame oil, safflower oil, castor oil, peanut oil, palm oil, and medium chain fatty acid triglycerides; preferably, the injectable oil is soybean oil.

Embodiment 9. The composition according to any one of Embodiments 1 to 8, wherein the phospholipid is selected from one or more of egg yolk lecithin, soybean phospholipid, hydrogenated soybean phospholipid, dierucoyl phosphatidylcholine, 1,2-dimyristoyl-sn-glycero-3-phosphocholine, phosphatidyl ethanolamine (Cephalin), phosphatidyl serine, phosphatidyl glycerol, diphosphatidyl glycerol (Cardiolipin), phosphatidyl inositol and sphingomyelin; preferably, the phospholipid is selected from one or more of egg yolk lecithin, soybean phospholipid, phosphatidyl ethanolamine and phosphatidyl serine; more preferably, the phospholipid is egg yolk lecithin.

Embodiment 10. The composition according to any one of Embodiments 1 to 9, wherein the low-molecular-weight alcohol is selected from one or more of ethanol, ethylene glycol, propylene glycol, glycerol, isopropanol, n-butanol, isobutanol and pentanol; preferably, the low-molecular-weight alcohol is ethanol.

Embodiment 11. The composition according to any one of Embodiments 1 to 10, wherein the injectable oil is soybean oil, the phospholipid is soybean lecithin, and the low-molecular-weight alcohol is ethanol, such as absolute ethanol.

Embodiment 12. The composition according to Embodiment 11, wherein the composition consists of:

| | |
|---|---|
| Docetaxel | 2.25% w/w |
| Soybean oil for injection | 11.25% w/w |
| Egg yolk lecithin | 33.74% w/w |
| Citric acid | 0.05%, 0.1% or 0.2% w/w |
| Ethanol | make up to 100% w/w. |

Embodiment 13. A method for preparing the composition according to any one of Embodiments 1 to 12, including:
(1) Dissolving docetaxel and citric acid in the low-molecular-weight alcohol, and then sequentially adding with the phospholipid and the injectable oil to form a uniform solution; and
(2) Sterilizing the uniform solution obtained in step (1) by filtration, and filling the solution into a container to give the docetaxel composition of the present disclosure.

Embodiment 14. A pharmaceutical product comprising the composition according to any one of Embodiments 1 to 12.

Embodiment 15. A docetaxel emulsion for injection comprising the composition according to any one of Embodiments 1 to 12 and an aqueous vehicle for injection, for example, selected from 5% glucose injection, 0.9% sodium chloride injection, glucose and sodium chloride injection, water for injection, or a mixture thereof, wherein the emulsion has an emulsion droplet size in the range of 200-700 nm.

Embodiment 16. Use of the composition according to any one of Embodiments 1 to 12 in the preparation of a medicament, particularly an emulsion, for the treatment of cancer.

Embodiment 17. A method for treating cancer comprising administering a therapeutically effective amount of the emulsion according to Embodiment 15 to a patient with cancer.

The technical features listed in the various embodiments as disclosed herein can be combined with each other to generate new technical solutions, which are all within the scope of the present disclosure.

### Advantages of the composition of the present disclosure

The inventors' studies showed that only when citric acid was present in a specific amount in the composition, the chemical stability of docetaxel could be improved, the self-emulsification to form the emulsion droplet size was not affected, and docetaxel did not crystallize out quickly.

The self-emulsifiable docetaxel composition for injection of the present disclosure has the following advantages:
1. The composition of the present disclosure has reduced adverse effects. The toxic and adverse effects of Tween-80 as excipient have been widely confirmed. For example, serious incidents, including deaths, have occurred many times with traditional Chinese medicine injections (such as Houttuynia cordata injection) containing Tween-80. Polyoxyethylene castor oil also has similar problems. In the composition of the present disclosure, phospholipid is used for replacing the solubilizer which generates allergic reactions, and excipients used in the composition are safe. Phospholipid is an important component of biological membranes in human body, and is a basic substance of life. Phospholipid is non-toxic and has good biocompatibility. As a drug carrier, it is easy to degrade in vivo and has no immunogenicity. Therefore, the present disclosure avoids the allergic reactions caused by Tween-80 and polyoxyethylene castor oil, and thus avoids the need of preventive use of dexamethasone before the administration of docetaxel, which greatly improves the compliance of patients in the clinical use.
2. Both the composition of the present disclosure and the emulsion formed by its dilution have good stability. The composition of the present disclosure is anhydrous solutions and has improved chemical stability. Moreover, compared with Taxotere^{®}, the docetaxel composition for injection of the present disclosure forms, upon being diluted to self-emulsify, an emulsion which remains stable for a longer time without rapid precipitation of drug crystals. Thus, the composition of the present disclosure possesses excellent chemical stability, and the emulsion resulting from its dilution exhibits superior physical stability.
3. The composition of the present disclosure is advantageous for industrial production. Compared with other docetaxel carrier systems under development (such as polymeric micelles, albumin nanoparticles, liposomes, lipid nanoparticles, submicroemulsions and the like), the composition of the present disclosure has a simpler preparation process (similar to a preparation method of a solution-type injection), does not require special instruments and equipment, is relatively lower in preparation cost, and is less prone to bring impurities during preparation. Therefore, their advantages for industrialization are significant.
4. The composition of the present disclosure has a better therapeutic effect than the commercially available preparations.

### Examples

The following examples aim to assist those skilled in the art in understanding the invention well, and should not be construed as limitation on the protection scope of the present disclosure.

### Examples 1-5

### 1. Components and amounts thereof in the compositions of Examples 1-5

**Table 1. Components and amounts thereof in the compositions of Examples 1-5**

| **Components** | **Example 1 (amount in g)** | **Example 2 (amount in g)** | **Example 3 (amount in g)** | **Example 4 (amount in g)** | **Example 5 (amount in g)** |
|---|---|---|---|---|---|
| Docetaxel | 2.25 | 2.25 | 2.25 | 2.25 | 2.25 |
| Soybean oil for injection | 11.25 | 11.25 | 11.25 | 11.25 | 11.25 |
| Egg yolk lecithin | 33.74 | 33.74 | 33.74 | 33.74 | 33.74 |
| Anhydrous citric acid | 0 | 0.1 | 0.05 | 0.025 | 0.0125 |
| Abosulte ethanol | Added to 100 | Added to 100 | Added to 100 | Added to 100 | Added to 100 |

### 2. Preparation method of the compositions of Examples 1-5

The docetaxel compositions of Examples 1-5 were prepared by:
(1) Dissolving the specified amount of docetaxel and anhydrous citric acid in the specified amount of absolute ethanol under stirring at room temperature;
(2) Adding the specified amount of egg yolk lecithin, and stirring to dissolve;
(3) Adding the specified amount of soybean oil, and stirring until uniform;
(4) Sterilizing by filtration, filling into injection vial, and capping.

### 3. Examination on the physicochemical properties of the compositions of Examples 1-5

### 3.1. Appearance and pH value determination of the compositions prepared in Examples 1-5:

The compositions of Examples 1-5 were all uniform, clear and pale-yellow solutions, and the formulations containing varying amounts of anhydrous citric acid were not significantly different in appearance.

15g of each of the composition of Example 1 containing no citric acid and the composition of Example 2 containing 0.1% citric acid was subjected to DGi 113-SC acid-base titration in non-aqueous media using a smart composite glass electrode (Mettler, Switzerland) at room temperature to measure the pH value. It was shown that the composition of Example 1 had a pH value of 5.82, and the composition of Example 2 had a pH value of 3.98.

### 3.2. Self-emulsification of the compositions prepared in Examples 1-5:

The compositions prepared in Examples 1-5 were respectively diluted with 5% glucose injection to a concentration of about 0.3mg/mL of docetaxel, mechanically shaken and then left for standing at room temperature. The droplet size in the formed emulsion was measured at 0h, 4h, 6h, 8h and 12h, respectively, and the precipitation of docetaxel crystals was observed.

It was observed that the compositions of Examples 1-5 all formed an off-white emulsion upon dilution. After standing at room temperature for 12 hours, no stratification or docetaxel crystallization was observed in any of the emulsions.

The results of the droplet size and drug precipitation of the emulsions are shown in Table 2.

**Table 2. Average droplet size in the emulsions prepared from the compositions of Examples 1-5**

| Example No. | 0h Average size nm | 4h Average size nm | 6h Average size nm | 8h Average size nm | 12 h Average size nm |
|---|---|---|---|---|---|
| Example 1 (containing no citric acid) | 399.6 | 409.4 | 373.8 | 385.4 | 387.6 |
| Example 2 (containing 0.1% citric acid) | 418.4 | 379.4 | 436.5 | 406.5 | 395.1 |
| Example 3 (containing 0.05% citric acid) | 486.8 | 454.2 | 395.1 | 397.6 | 402.3 |
| Example 4 (containing 0.025% citric acid) | 435.4 | 441.5 | 359.7 | 388.4 | 396.7 |
| Example 5 (containing 0.0125% citric acid) | 420.8 | 393.5 | 435.0 | 387.2 | 411.3 |

The results in Table 2 showed that after the compositions of Examples 1-5 were diluted into emulsions, the droplet size of the emulsions changed over time, but no stratification or docetaxel crystallization occurred, and the droplet size of the emulsions was within the range acceptable for injection.

### 3.3. Stability Test 1

The above five compositions were respectively heated at 100°C for 30min. The appearance of the formulations before and after heating was observed, and the content of docetaxel and the change in the related substances were determined. If precipitation occurred and/or the content of docetaxel exceeded the limit of 90.0%-110.0% specified by USP 43 for Docetaxel Injection, the formulation was judged unstable.

After heating, the composition of Example 1 without citric acid became cloudy and dark brown, with precipitation setting at the bottom of the bottle. The compositions of Examples 2-5 remained clear, and changed from yellow to light brown without precipitation. The change in docetaxel content after heating the composition at 100°C for 30 minutes is shown in Table 3.

**Table 3. Contents of docetaxel in the compositions before and after heating at 100°C for 30 minutes**

| Example No. | Content before heating | Content after heating | Decrease in content |
|---|---|---|---|
| Example 1 (containing no citric acid) | 100.40% | 96.27% | 4.13% |
| Example 2 (containing 0.1% citric acid) | 100.81% | 99.75% | 1.07% |
| Example 3 (containing 0.05% citric acid) | 100.95% | 99.22% | 1.73% |
| Example 4 (containing 0.025% citric acid) | 100.40% | 97.14% | 3.26% |
| Example 5 (containing 0.0125% citric acid) | 100.04% | 96.29% | 3.75% |

The results in Table 3 showed that the composition of Example 1, which does not contain citric acid, not only exhibited precipitation after heating, but also experienced the greatest decrease in docetaxel content. In contrast, the addition of citric acid to the formulations reduced the percentage decrease in docetaxel content, with the composition of Example 2 containing 0.1% citric acid exhibiting the least decrease in docetaxel content.

According to USP 43, the total impurities in Docetaxel Injection shall not exceed 3.5%, and the limit for Impurity e among these impurities shall not exceed 1.0%.

Therefore, the contents of the total impurities and Impurity e of the compositions of each Example were further examined after heating at 100°C for 30 minutes. The results showed that the content of the total impurities was less than 3.5% for all compositions, and the content of Impurity e was shown in Table 4.

**Table 4. Content of Impurity e in the compositions before and after heating at 100°C for 30 minutes**

| Example No. | Content of Impurity e (%) before heating | Content of Impurity e (%) after heating |
|---|---|---|
| Example 1 (containing no citric acid) | 0 | 2.11 |
| Example 2 (containing 0.1% citric acid) | 0 | 0.54 |
| Example 3 (containing 0.05% citric acid) | 0 | 0.85 |
| Example 4 (containing 0.025% citric acid) | 0 | 1.24 |
| Example 5 (containing 0.0125% citric acid) | 0 | 1.63 |

The results in Table 4 showed that when citric acid was added in the amount of 0%, 0.0125%, and 0.025%, the content of Impurity e exceeded 1.0%, failing to meet the requirements of USP 43. However, when citric acid was added in the amount of 0.05% and 0.1%, the content of Impurity e was within the specified limit. Moreover, the droplet size of the emulsions formed by diluting these two compositions remained within the range of 300 nm-500 nm after heating, showing no significant change compared with that before heating.

### 3.4. Stability Test 2

The composition of Example 1 (containing no citric acid) and the composition of Example 2 (containing 0.1% citric acid) were subjected to stability test at an elevated temperature of 40°C or under intense light exposure for sampling after 5 days and 10 days to test the related substances and the docetaxel content. The results are shown in Table 5.

**Table 5. Stability of the compositions of Examples 1 and 2 at 40°C or under light**

| **Test Item** | **Limit specified by USP 43** | **Day 0** | | **40°C** | | | | **5500±500Lux, 100µw** | | | |
|---|---|---|---|---|---|---|---|---|---|---|---|
| | | Example 2 | Example 1 | Day 5 Example 2 | Day 5 Example 1 | Day 10 Example 2 | Day 10 Example 1 | Day 5 Example 2 | Day 5 Example 1 | Day 10 Example 2 | Day 10 Example 1 |
| **Related Substances (%)** | Impurity a ≤0.30% | 0.014 | 0.000 | 0.0026 | 0.000 | 0.0038 | 0.000 | 0.0043 | 0.166 | 0.0052 | 0.101 |
| | Impurity b ≤0.20% | 0.021 | 0.092 | 0.021 | 0.106 | 0.021 | 0.119 | 0.016 | 0.081 | 0.017 | 0.075 |
| | Impurity c ≤1.3% | 0.000 | 0.000 | 0.000 | 0.000 | 0.000 | 0.000 | 0.000 | 0.000 | 0.000 | 0.053 |
| | Impurity d ≤1.5% | 0.015 | 0.000 | 0.0096 | 0.000 | 0.034 | 0.000 | 0.018 | 0.000 | 0.018 | 0.000 |
| | Impurity e ≤1.0% | 0.042 | 0.043 | 0.059 | 0.267 | 0.081 | 0.364 | 0.048 | 0.171 | 0.050 | 0.185 |
| | Impurity f ≤0.5% | 0.000 | 0.000 | 0.000 | 0.000 | 0.000 | 0.000 | 0.000 | 0.000 | 0.000 | 0.000 |
| | Any other single largest impurity ≤0.2% | 0.052 | 0.145 | 0.047 | 0.189 | 0.049 | 0.189 | 0.047 | 0.292 | 0.048 | 0.289 |
| | Total impurities ≤3.5% | 0.22 | 0.407 | 0.24 | 0.780 | 0.29 | 0.790 | 0.23 | 1.303 | 0.24 | 1.301 |
| **Content** | 90.0%-110% of the labeled amount | 100.2 | 100.3 | 99.6 | 98.3 | 99.8 | 98.2 | 100.3 | 97.3% | 99.1 | 97.1 |

| | | | | | | | | | | | |
|---|---|---|---|---|---|---|---|---|---|---|---|
| Impurity a is 10-deacetylbaccatin, Impurity b is 2-debenzoxyl 2-pentenyl docetaxel, Impurity c is crotonaldehyde analog, Impurity d is 6-oxodocetaxel, Impurity e is 4-epidocetaxel, and Impurity f is 4-epi-6-oxodocetaxel. | | | | | | | | | | | |

As shown in the table above, the composition of Example 1 containing no citric acid failed to meet the requirements of USP 43 for the level of other single largest impurity after 5 or 10 days of exposure to light. In contrast, the composition of Example 2 met all requirements of USP 43. Furthermore, after exposure to 40°C or intense light, the levels of Impurity e and total impurities in the composition of Example 2 containing 0.1% citric acid were significantly lower than those in the composition of Example 1 containing no citric acid. More specifically, after 10 days at 40°C or under light, Impurity e and total impurities in the composition of Example 2 containing 0.1% citric acid remained substantially unchanged, whereas the composition of Example 1 containing no citric acid had approximately 4-fold increase for the level of Impurity e and 3-fold increase for the level of total impurities, respectively.

### 3.5. Stability Test 3

The composition of Example 1 (containing no citric acid) and the composition of Example 2 (containing 0.1% citric acid) were left at 25°C ± 2°C and 60% ± 5% relative humility (RH) for 2 months, and tested for the related substances and the docetaxel content. The results are shown in Table 6.

**Table 6. Stability of the compositions of Example 1 and 2 at 25°C ± 2°C and 60% ± 5% RH**

| **Test Item** | **Limit specified by USP 43** | **Time** | | | |
|---|---|---|---|---|---|
| | | 0 month | | 2 months | |
| | | Example 2 | Example 1 | Example 2 | Example 1 |
| **Related Substances** | single largest impurity ≤0.2% | 0.052 | 0.049 | 0.054 | 0.285 |
| | Total impurities≤3.5% | 0.16 | 0.192 | 0.40 | 0.741 |
| **Content** | 90.0%-110% of the labeled amount | 101.2 | 99.3 | 101.4 | 97.2 |

As shown in the table above, the level of the single largest impurity in the composition of Example 1 containing no citric acid exceeded the specified limit after the composition was stored for 2 months at 25°C ± 2°C and 60% ± 5% RH. The increases of the single largest impurity and the total impurities in the composition of Example 2 containing 0.1% citric acid after storage for 2 months under the same condition were significantly lower than those in the composition of Example 1 containing no citric acid.

### Examples 6-8

**Table 7. Components and amounts thereof in the compositions of Examples 6-7**

| **Components** | **Example 6 (amount in g)** | **Example 7 (amount in g)** | **Example 8 (amount in g)** |
|---|---|---|---|
| Docetaxel | 2.25 | 2.25 | 2.25 |
| Soybean oil for injection | 11.25 | 11.25 | 11.25 |
| Egg yolk lecithin | 33.74 | 33.74 | 33.74 |
| Anhydrous citric acid | 0.4 | 0.8 | 1.5 |
| Absolute ethanol | Added to 100 | Added to 100 | Added to 100 |

The preparation method used in Examples 6-8 was the same as that used in Examples 1-5.

The compositions of Examples 6-8 were all uniform, clear and pale-yellow solutions, and were not significantly different in appearance from the composition of Example 1.

Self-emulsification of the compositions prepared in Examples 6-8: the compositions prepared in Examples 6 to 8 were diluted with 5% glucose injection respectively to a docetaxel concentration of about 0.3mg/mL, mechanically shaken and then left for standing at room temperature. The droplet size in the formed emulsion was measured at 0h, 4h, 6h, 8h and 12h, respectively, and the precipitation of docetaxel crystals was observed.

It was observed that the compositions of Examples 6-8 all formed an off-white emulsion upon dilution, with the emulsion droplet sizes all being more than 700nm as shown in Table 8. After standing at room temperature, docetaxel crystals precipitated rapidly from the emulsions formed from the dilution of the compositions of Examples 6-8.

**Table 8. Droplet sizes and docetaxel crystallization in the emulsions prepared from the compositions of Examples 6-8**

| | 0h | 4h | 6h | 8h | 12h |
|---|---|---|---|---|---|
| Example No. | Average size nm | Average size nm | Average size nm | Average size nm | Average size nm |
| Example 1 (containing no citric acid) | 390.0 | 409.4 | 373.8 | 385.4 | 387.6 |
| Example 6 (containing 0.4% citric acid) | 1077.0 | - | - | - | - |
| Example 7 (containing 0.8% citric acid) | 739.5 | - | - | - | - |
| Example 8 (containing 1.5% citric acid) | 946.8 | - | - | - | - |

| | | | | | |
|---|---|---|---|---|---|
| Notes: - represents docetaxel crystallization | | | | | |

It can be seen that the compositions containing 0.4%, 0.8% and 1.5% anhydrous citric acid all formed emulsion with droplet size of more than 700 nm after self-emulsification (the inventors have found that when an emulsion has a droplet size of more than 700 nm, the emulsion is prone to stratify and cannot remain stable), indicating that the compositions have poor self-emulsification ability. Moreover, docetaxel crystals precipitated when the emulsions stood for 4 hours, showing that the formed emulsions had poor physical stability.

Since emulsions were physically unstable, no chemical stability studies were performed on these compositions.

### Example 9

**Table 9. Components and amounts thereof in the composition of Example 9**

| **Components** | **Amount in g** |
|---|---|
| Docetaxel | 2.25 |
| Soybean oil for injection | 11.25 |
| Egg yolk lecithin | 33.74 |
| Anhydrous citric acid | 0.2 |
| Absolute ethanol | Added to 100 |

The preparation method used in Example 9 was the same as that used in Examples 1-5.

The composition of Example 9 was a uniform, clear and pale-yellow solution, and was not significantly different in appearance from the compositions of Examples 1-5.

Self-emulsification of the composition prepared in Example 9: the composition prepared in Example 9 was diluted with 5% glucose injection to a docetaxel concentration of about 0.3mg/mL, mechanically shaken and then left for standing at room temperature. The droplet size in the formed emulsion was measured at 0h, 4h, 6h, 8h and 12h, respectively, and the precipitation of docetaxel crystals was observed.

It was observed that the composition of Example 9 formed an off-white emulsion upon dilution. After standing at room temperature for 12h, no stratification or docetaxel crystallization was observed.

**Table 10. Average droplet size of the emulsion prepared from the composition of Example 9**

| Example No. | 0h Average size nm | 4h Average size nm | 6h Average size nm | 8h Average size nm | 12 h Average size nm |
|---|---|---|---|---|---|
| Example 9 (containing 0.2% citric acid) | 520.9 | 538.2 | 686.3 | 657.3 | 662.4 |

After heating at 100°C for 30 minutes, the composition of Example 9 containing 0.2% citric acid changed from yellow to light brown, no precipitation occurred, and the determined contents of docetaxel and Impurity e are shown in Tables 11 and 12.

**Table 11. Content of docetaxel in the composition of Example 9 before and after hearing at 100°C for 30 minutes**

| **Example No.** | **Content before heating** | **Content after heating** | **Decrease in content** |
|---|---|---|---|
| Example 9 (containing 0.2% citric acid) | 100.19% | 99.04 percent | 1.15% |

**Table 12. Content of Impurity e in the composition of Example 9 before and after hearing at 100°C for 30 minutes**

| **Example No.** | Content of Impurity e (%) | Content of Impurity e (%) |
|---|---|---|
| Example 9 (containing 0.2% citric acid) | 0 | 0.32 |

After heating at 100°C for 30 minutes, the composition of Example 9 has the total impurities of less than 3.5%, which met the requirements of USP 43. The contents of docetaxel and Impurity e also met the requirements of USP 43.

Based on the experimental data in the above Examples, the following conclusions can be drawn:
The amount of citric acid affects the initial droplet size of the emulsion formed upon dilution and crystallization upon standing. Thus, the amount of citric acid affects the self-emulsifying ability of the composition of the present disclosure and the physical stability of the emulsion formed upon dilution. When the amount of citric acid is higher, it results in poor self-emulsifying ability of the composition and poor physical stability of the formed emulsion. In the meantime, the amount of citric acid also affects the chemical stability of the composition prepared. When the amount of citric acid is lower, the content of impurities in the composition upon standing exceeds the limit specified by USP 43. Therefore, in order to achieve both physical and chemical stability, the amount of citric acid used must be kept within a specific range.

Based on the investigation of the above nine examples, it is found that when the amount of citric acid in the composition ranges from 0.05% to 0.2%, it effectively balances both the chemical stability of docetaxel (the content of docetaxel and the related substances meet the requirements of USP 43 under the stress conditions) and the physical stability of the emulsion formed by self-emulsification (the emulsion formed upon dilution of the composition does not show stratification and docetaxel crystallization after standing at room temperature for 12 hours). When the amount of the anhydrous citric acid used is 0.1%, the balance is optimal. Consequently, the content remains stable, related substances increase minimally, the droplet size of emulsion upon self-emulsification is not more than 500nm, and no stratification or docetaxel crystallization occurs in the emulsion.

### Examples 10-13

### Study on the amount of injectable oil

**Table 13. Components and amounts thereof in the compositions of Examples 10-13**

| **Components** | **Example 10 (amount in g)** | **Example 11 (amount in g)** | **Example 12 (amount in g)** | **Example 13 (amount in g)** |
|---|---|---|---|---|
| Docetaxel | 2.25 | 2.25 | 2.25 | 2.25 |
| Soybean oil for injection | 22.50 | 4.50 | 15 | 6 |
| Egg yolk lecithin | 33.74 | 33.74 | 33.74 | 33.74 |
| Anhydrous citric acid | 0.1 | 0.1 | 0.1 | 0.1 |
| Absolute ethanol | Added to 100 | Added to 100 | Added to 100 | Added to 100 |

The preparation method used in Examples 10-13 was the same as that used in Examples 1-5.

The compositions of Examples 10, 11, 12, 13 were all uniform, clear and pale-yellow solutions, and the formulations containing different amounts of soybean oil were not significantly different in appearance.

Self-emulsification of the compositions prepared in Examples 10-13: the compositions prepared in Examples 10-13 were diluted with 5% glucose injection to a docetaxel concentration of about 0.3mg/mL, respectively, and mechanically shaken, whereupon visible oil droplets were immediately apparent in the emulsion formed by the composition of Example 10, and fine floccules occurred in the emulsion formed by the composition of Example 11, while the emulsions formed by the compositions of Examples 12 and 13 showed neither oil droplets or fine floccules, nor did they exhibit docetaxel crystallization after standing at room temperature for 12 hours.

### Examples 14-17

### Investigation of the amount of phospholipid

**Table 14. Components and amounts thereof in the compositions of Examples 14-17**

| **Components** | **Example 14 (amount in g)** | **Example 15 (amount in g)** | **Example 16 (amount in g)** | **Example 17 (amount in g)** |
|---|---|---|---|---|
| Docetaxel | 2.25 | 2.25 | 2.25 | 2.25 |
| Soybean oil for injection | 11.25 | 11.25 | 11.25 | 11.25 |
| Egg yolk lecithin | 45 | 20 | 40 | 25 |
| Anhydrous citric acid | 0.1 | 0.1 | 0.1 | 0.1 |
| Absolute ethanol | Added to 100 | Added to 100 | Added to 100 | Added to 100 |

The preparation method used in Examples 14-17 was the same as that used in Examples 1-5.

The compositions of Examples 14, 15, 16, 17 were all uniform, clear and pale-yellow solutions, and the formulations containing different amounts of phospholipid were not significantly different in appearance.

Self-emulsification of the compositions prepared in Examples 14-17: the compositions prepared in Examples 14-17 were diluted with 5% glucose injection to a docetaxel concentration of about 0.3mg/mL, respectively, and mechanically shaken for observation. Upon drawing the emulsion formed by the composition of Example 14, the emulsion was found to be particularly viscous, while the emulsion formed by the composition of Example 16, although slightly viscous, was significantly better than the emulsion formed by the composition of Example 14. After mechanical shaking, visible fine floccules occurred in the emulsion formed by the composition of Example 14, and docetaxel crystals principiated in the emulsion formed by the composition of Example 15. In contrast, the emulsions formed by the compositions of Examples 16 and 17 showed neither oil droplets or fine floccules, nor did they exhibit docetaxel crystallization after standing at room temperature for 12 hours.

### Example 18 - Allergy Test

The composition of Example 2 of the present disclosure (self-emulsifiable drug delivery system, SEDDS), Taxotere^{®} and ethanol-free docetaxel injection of Eagle Pharmaceuticals, Inc. (hereinafter referred to as "Eagle") were diluted to 4 mg/ml with 5% glucose injection, and tested for allergy in Hartley guinea pigs (SPF grade, male, 300-400g, purchased from Beijing Charles River Laboratory Animal Center): a clinically equivalent dose (11.035 mg/kg) was administered via a single intra-tarsal vein injection of different formulations in three test groups (n=3/group) and one positive control group (10% Tween-80, n=3/group). The behavior of the guinea pigs was recorded immediately after dosing, and the results are as shown in Table 15.

**Table 15. Behavioral Scoring of guinea pigs after intravenous administration of the formulations**

| Group | No. | Normal (0) | Restlessness, bristling hair, trembling, scratching the nose (1) | Shortness of breath, urination, tearing (2) | Shortness of breath, unsteady gait, jumping, rotating (3) | Grading |
|---|---|---|---|---|---|---|
| Positive control group, 10% Tween-80 | 1 | - | - | Shortness of breath | unsteady gait | strongly positive |
| | 2 | - | trembling | tearing | shortness of breath | |
| | 3 | - | trembling, scratching the nose | Shortness of breath | rotating | |
| Formulation of Example 2 | 1 | normal | - | - | - | negative |
| | 2 | normal | - | - | - | |
| | 3 | normal | - | - | - | |
| Commercially available Taxotere^{®} containing Tween-80 | 1 | - | trembling, scratching the nose | urination | jumping, rotating | strongly positive |
| | 2 | - | trembling, scratching the nose | - | unsteady gait | |
| | 3 | - | trembling | urination | jumping, rotating | |
| Commercially available Eagle containing Tween-80 | 1 | - | trembling, scratching the nose | - | jumping | strongly positive |
| | 2 | - | trembling, scratching the nose | - | jumping | |
| | 3 | - | trembling | tearing | unsteady gait | |

The results showed that both the formulations containing Tween-80 (Taxotere^{®} and Eagle) and the positive control Tween-80 caused significant allergic behaviors in guinea pigs, including restlessness, trembling, itching, tearing, shortness of breath, unsteady gait and rotating, with the overall behavioral changes including increased activity, from jumping and rotating to gradually unsteady gait, and listlessness. However, the guinea pigs administered with the composition of Example 2 without Tween-80 did not show the above abnormal behavioral responses.

### Example 19 - Anticancer Activity Test

### 1. Cytotoxicity Test:

4T-1 cells were cultured in a 25 cm² flask at 37°C in 5% CO₂ using DMEM medium supplemented with 10% FBS and 1% dual-antibiotics (penicillin and streptomycin). The cells were passaged when they reached 70% confluence in the flask, with a passage interval of 1-2 days and ad dilution ratio of 1:6. During passaging, 3 ml of complete medium was first added to wash the cells, then 1 ml of 0.25% trypsin was added to digest the cells for 1-1.5 minutes (37°C). The cells were observed to round up under a microscope. The bottom of the culture plate was gently tapped, and if more than half of the cells floated, 4-5 ml of complete medium was added immediately to terminate digestion. The cells were collected into a 15-ml centrifuge tube, and centrifuged at 1000 rpm for 3min at room temperature to pellet the cells. After centrifuging, the supernatant was separated and added with 6 ml of complete medium to resuspend the cells, and mixed uniformly. 1 ml of cell suspension was taken and added into a 25 cm² culture flask filled with 5ml of complete medium, avoiding bubbles. After shaking to disperse uniformly, the flask was placed in a 37°C and 5% CO₂ incubator for continued culture.

When 4T-1 cells reached the logarithmic growth phase, they were digested with 0.25% trypsin and resuspended in 6ml of complete medium. The cells were counted and adjusted to a cell concentration of 2×10⁴ cells/ml. 100 µL of the suspension was evenly seeded onto a 96-well plate, filling the marginal wells with sterile PBS. The plate was incubated in an incubator at 5% CO₂ and 37°C for 12-24 hours. When the cells grew to about 10% confluence, the formulations containing the drug in gradient concentrations were added. There were three test groups and one control group. The cells in the test groups were incubated with the formulations obtained by diluting the composition of Example 2, Taxotere^{®} and Eagle with 5% glucose injection, and the cells in the control group were incubated with complete medium without drug. The gradient drug concentrations for the test groups were 5, 1, 0.2, 0.04, 0.008, 0.0016, 0.00032, 0.000064, 0.0000064, 0 µg/ml. Meanwhile, a blank excipient control group at the highest concentration was set to confirm that the excipient at the highest concentration was not toxic. In addition, a blank group without cells but supplemented with CCK-8 complete medium was set as the baseline. After administration, the 96-well plate was placed in a 37°C isothermal incubator for further incubation. After 48-72 hours until the cells grew to confluence, the 96-well plate was removed, the drug-containing culture liquid was sucked out of the plate, and a 100 µl CCK-8 working solution (10-fold dilution of 10 µl CCK-8 with complete medium) was added into each well for incubating at 37°C in dark for 1-4 h. The absorbance of each well was measured using an an enzyme-linked immunosorbent assay (ELISA) reader at 450 nm. Cell viability = (OD value of test well - OD value of blank well) / (OD value of control well - OD value of blank well) × 100%. A sigmoidal curve of viability versus concentration was fitted using Graphpad to give half maximal inhibitory concentration (IC₅₀) value.

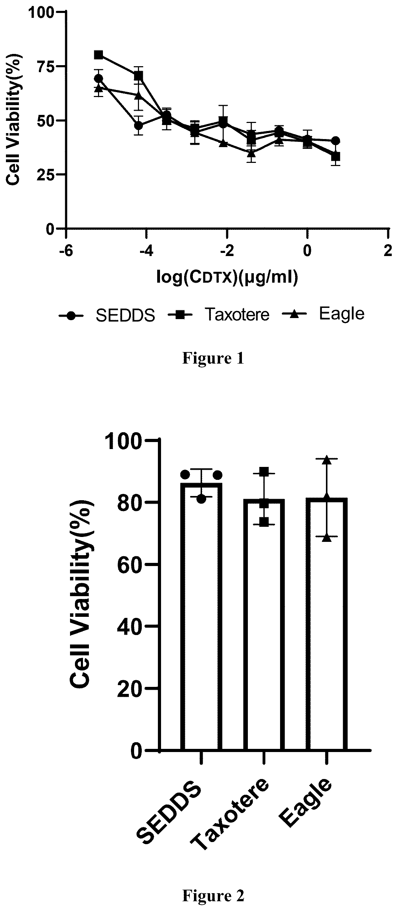

The cell viability of each well was assessed using the CCK-8 assay kit. A cell viability curve was plotted against the logarithm of drug concentration, as shown in Figure 1. It can be seen from Figure 1 that the change trends of the cell viability of the three formulations are substantially similar. Particularly, the cell viability after incubation at a concentration in the range of 0.32 ng/ml to 5 µg/ml was all less than 50% for the three formulations, and there was not significant difference between groups.

The blank formulation control group at the highest concentration (the blank formulation control for Example 2: 11.25 mg soybean oil for injection, 33.74mg egg yolk lecithin, 0.1 mg anhydrous citric acid, absolute ethanol added to 100 mg; the blank formulation control for Taxotere^{®}: 0.5 ml Tween-80, 1.5 ml 13% w/w aqueous ethanol; the blank formulation control for Eagle: 27.5 mg soybean oil, 585.0 mg Tween-80, 10.2 mg citric acid, 442.2 mg PEG300) showed cell viability as shown in Figure 2 after incubation with the same number of cells for 48 h. It can be seen that the blank excipients of the three formulations did not have proliferative toxicity to 4T1 cells, and the cell viability was all above 80%, proving that the cell proliferative toxicity brought by the complete formulation was completely produced by the active drug docetaxel.

### 2. Animal Test:

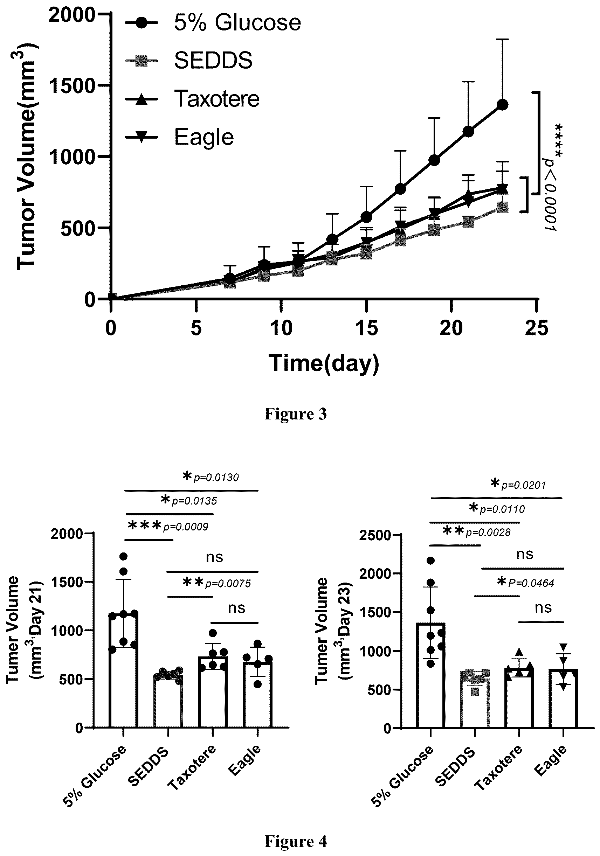

When the 4T1 cells grew to reach a confluency of above 80%, the cells were digested with 0.25% trypsin, centrifuged to collect cell pellet, washed approximately three times by adding with FBS-free DMEM medium to resuspend, and finally resuspended in a serum-free DMEM culture medium for counting. The cells were diluted appropriately with FBS-free DMEM medium to a cell concentration of 10⁷ cells/ml. 100 µl of the cell suspension was inoculated subcutaneously into the right axilla of BALB/C mice. Approximately 5-7 days after subcutaneous inoculation of the 4T1 cells, tumor volumes reached to about 100 mm³-150 mm³, and the BALB/C mice (female, 18-20g, purchased from the Laboratory Animals Center, Department of Medicine, Peking University) were randomly divided into four groups (n = 6): three test groups (SEDDS, Taxotere^{®}, Eagle), and one blank control group (5% glucose injection). The mice were injected intravenously with 6 mg/kg of the different formulations (dilution with 5% glucose injection) every other day for a total of four injections. The changes of body weight and tumor volume (V = (length × width²)/2) of the mice were recorded, and the results are as shown in Figure 3. As depicted in Figure 3, the increase of the tumor volume in the glucose control group significantly accelerated from the fourth day after the last administration (Day 11), and the average tumor volume reached above 1000 mm³ by Day 19. However, the increases of the tumor volume in the three test groups were relatively flat, and the average tumor volume did not exceed 1000 mm³ on Day 23 (the end point of the experiments), showing that the taxane drug docetaxel significantly inhibited the growth of 4T1 subcutaneous tumors. Although the tumor growth was significantly inhibited in all the three test groups, the tumor of the SEDDS test group (the formulation of Example 2) grew more slowly than those of the Taxotere^{®} and Eagle groups, with a significant difference in tumor volume on Day 21 (p = 0.0075) and on Day 23 (p = 0.0464) compared with the other two test groups, as shown in Figure 4.

### Brief Description of Drawings

Figure 1 shows the cell viability curves produced by in the three formulations in the "Cytotoxicity Test" of Example 19, wherein the abscissa log (C_{DTX}) represents the logarithm of docetaxel concentration in µg/ml, the ordinate represents the percentage of cell survival, SEDDS denotes the diluted formulation of the composition of Example 2 of the present application, Taxotere denotes the diluted formulation of the docetaxel injection sold under the trade name Taxotere^{®} manufactured by Sanofi-Aventis, and Eagle denotes the diluted formulation of the ethanol-free docetaxel injection manufactured by Eagle Pharmaceuticals, Inc. The results in Figure 1 show that there is no significant difference in the inhibitory effect of the three formulations on the cell growth.
Figure 2 shows the cell viability produced by the blank formulations prepared from the excipients used in Taxotere^{®}, the ethanol-free docetaxel injection manufactured by Eagle Pharmaceuticals, Inc. and the composition of Example 2 (SEDDS) in the "Cytotoxicity Test" of Example 19, wherein the ordinate represents the percentage of cell survival, SEDDS denotes the blank formulation prepared from the excipients used in the composition of Example 2 of the present application, Taxotere denotes the blank formulation prepared from the excipients used in the docetaxel injection sold under the trade name Taxotere^{®} manufactured by Sanofi-Aventis, and Eagle denotes the blank formulation prepared form the excipients used in the ethanol-free docetaxel injection manufactured by Eagle Pharmaceuticals, Inc. The results in Figure 2 show that the excipients used in all the three formulations do not have inhibitory effect on cell growth.
Figure 3 shows the tumor growth curves (n=6) of 4T1 subcutaneously tumor-bearing mice treated with the three formulations in the "Animal Test" of Example 19, wherein the abscissa represents days, with the day of initial administration as Day 1, the ordinate represents the tumor growth volume (in mm³), SEDDS denotes the diluted formulation of the composition of Example 2 of the present application, Taxotere denotes the diluted formulation of the docetaxel injection sold under the trade name Taxotere^{®} manufactured by Sanofi-Aventis, and Eagle denotes the diluted formulation of the ethanol-free docetaxel injection manufactured by Eagle Pharmaceuticals, Inc. The results in Figure 3 show that the composition of Example 2 of the present application has better inhibitory effect on tumor.
Figure 4 shows the tumor volume on Day 21 (the left) and Day 23 (the right) of the 4T1 subcutaneous tumor-bearing mice treated with the three formulations in the "Animal Test" of Example 19, wherein the ordinate represents the tumor volume (in mm³), SEDDS denotes the diluted formulation of the composition of Example 2 of the present application, Taxotere denotes the diluted formulation of the docetaxel injection sold under the trade name Taxotere^{®} manufactured by Sanofi-Aventis, Eagle denotes the diluted formulation of the ethanol-free docetaxel injection manufactured by Eagle Pharmaceuticals, Inc., and "ns" indicates no significant difference. The results in Figure 4 show that the composition of Example 2 of the present application has better inhibitory effect on tumor.

## Claims

1. A self-emulsifiable docetaxel composition for injection, consisting of docetaxel, an injectable oil, a phospholipid, a low-molecular-weight alcohol and citric acid, wherein citric acid accounts for 0.05%-0.2% w/w based on the total weight of the composition.

2. The composition according to claim 1, wherein the amounts of docetaxel, the injectable oil, the phospholipid, the low-molecular-weight alcohol and citric acid in the composition are as follows:
| | |
|---|---|
| Docetaxel | 0.1%-5% w/w |
| Injectable oil | 5%-20% w/w |
| Phospholipid | 25%-40% w/w |
| Citric acid | 0.05%-0.2% w/w |
| Low-molecular-weight alcohol | make up to 100% w/w. |

3. The composition according to claim 1 or 2, wherein docetaxel accounts for 1%-3.5% w/w, for example, 1.5%-3.5% w/w, 1.5%-3% w/w, 2%-2.5% w/w, based on the total weight of the composition.

4. The composition according to any one of claims 1 to 3, wherein the injectable oil accounts for 6%-15% w/w based on the total weight of the composition.

5. The composition according to claim 1, wherein the phospholipid accounts for 25%-40% w/w based on the total weight of the composition.

6. The composition according to any one of claims 1 to 5, wherein citric acid accounts for 0.05%-0.1% w/w, preferably 0.1% w/w, based on the total weight of the composition.

7. The composition according to claim 1, wherein the composition consists of:
| | |
|---|---|
| Docetaxel | 1%-3.5% w/w |
| Injectable oil | 6%-15% w/w |
| Phospholipid | 25%-40% w/w |
| Citric acid | 0.05%-0.1% w/w |
| Low-molecular-weight alcohol | make up to 100% w/w. |

8. The composition according to any one of claims 1 to 7, wherein the injectable oil is selected from one or more of soybean oil, fish oil, olive oil, tea oil, oleic acid, sesame oil, safflower oil, castor oil, peanut oil, palm oil, and medium chain fatty acid triglycerides; preferably, the injectable oil is soybean oil.

9. The composition according to any one of claims 1 to 8, wherein the phospholipid is selected from one or more of egg yolk lecithin, soybean phospholipid, hydrogenated soybean phospholipid, dierucoyl phosphatidylcholine, 1,2-dimyristoyl-sn-glycero-3-phosphocholine, phosphatidyl ethanolamine (Cephalin), phosphatidyl serine, phosphatidyl glycerol, diphosphatidyl glycerol (Cardiolipin), phosphatidyl inositol and sphingomyelin; preferably, the phospholipid is selected from one or more of egg yolk lecithin, soybean phospholipid, phosphatidyl ethanolamine and phosphatidyl serine; more preferably, the phospholipid is egg yolk lecithin.

10. The composition according to any one of claims 1 to 9, wherein the low-molecular-weight alcohol is selected from one or more of ethanol, ethylene glycol, propylene glycol, glycerol, isopropanol, n-butanol, isobutanol and pentanol; preferably, the low-molecular-weight alcohol is ethanol.

11. The composition according to any one of claims 1 to 10, wherein the injectable oil is soybean oil, the phospholipid is soybean lecithin, and the low-molecular-weight alcohol is ethanol, such as absolute ethanol.

12. The composition according to claim 11, wherein the composition consists of:
| | |
|---|---|
| Docetaxel | 2.25% w/w |
| Soybean oil for injection | 11.25% w/w |
| Egg yolk lecithin | 33.74% w/w |
| Citric acid | 0.05%, 0.1% or 0.2% w/w |
| Ethanol | make up to 100% w/w. |

13. A method for preparing the composition according to any one of claims 1 to 12, including:
(1) Dissolving docetaxel and citric acid in the low-molecular-weight alcohol, and then sequentially adding with the phospholipid and the injectable oil to form a uniform solution; and
(2) Sterilizing the uniform solution obtained in step (1) by filtration, and filling the solution into a container to give the docetaxel composition of the present disclosure.

14. A pharmaceutical product comprising the composition according to any one of claims 1 to 12.

15. A docetaxel emulsion for injection comprising the composition according to any one of claims 1 to 12 and an aqueous vehicle for injection, for example, selected from 5% glucose injection, 0.9% sodium chloride injection, glucose and sodium chloride injection, water for injection, or a mixture thereof, wherein the emulsion has an emulsion droplet size in the range of 200-700 nm.

16. Use of the composition according to any one of claims 1 to 12 in the preparation of a medicament, particularly an emulsion, for the treatment of cancer.

17. A method for treating cancer comprising administering a therapeutically effective amount of the emulsion according to claim 15 to a patient with cancer.
